Europäisches Patentamt

European Patent Office

Office européen des brevets

(11) Publication number: **0 614 988 A1**

## EUROPEAN PATENT APPLICATION

(21) Application number: 94102319.4

(22) Date of filing: 16.02.94

(51) Int. Cl.5: **C12Q 1/68**, C12N 15/11, C12Q 1/70

The applicant has subsequently filed a sequence listing and declared, that it includes no new matter.

(30) Priority: 24.02.93 EP 93102844

(43) Date of publication of application:
14.09.94 Bulletin 94/37

(84) Designated Contracting States:
AT BE CH DE DK ES FR GB GR IE IT LI LU MC NL PT SE

(71) Applicant: **HOECHST AKTIENGESELLSCHAFT
Brüningstrasse 50
D-65929 Frankfurt am Main (DE)**

(72) Inventor: **Sczakiel, Georg, Dr.
Beintweg 9a
D-69181 Leimen (DE)**
Inventor: **Rittner, Karola
Kolpingstrasse 21
D-76694 Forst (DE)**

(54) Method of identifying fast-hybridizing single-stranded polynucleotides.

(57) The invention relates to an assay for in vitro selection of fast-hydridizing single-stranded polynucleotides applicable as inhibitor of pathogens, regulating physiological processes or as a diagnostic agent.

Rank Xerox (UK) Business Services
(3.10/3.09/3.3.4)

The present invention relates in general to a new method of identifying fast-hybridizing single-stranded polynucleotides.

Antisense nucleic acid-mediated control of gene expression and viral replication plays an increasingly significant role in vivo and in tissue culture cells. Two classes of antisense nucleic acids which differ in the specific form of application to living cells have been used. Relatively short synthetic and in many cases chemically modified antisense oligonucleotides have been applied successfully as well as long antisense RNAs synthesized in vitro or, in most cases, expressed intracellularly from antisense genes. Despite the great number of phenomenological descriptions of convincing biological effects obtained with antisense nucleic acids, unequivocal experimental evidence for the antisense principle has not been reported as frequently. In particular for eucaryotic antisense RNAs, systematic mechanistic analyses of double strand formation with target RNA in vitro and in vivo are lacking. In contrast to this, prokaryotic antisense RNA-regulated examples have been studied in more detail. For the antisense RNA-regulated plasmid copy number in E. coli it has been shown that the kinetic behaviour of antisense RNA in vitro is in agreement with the effectiveness in vivo (Tomizawa, J.I. and Som, T (1984) Cell **38**, 871-878). For replication of plasmid R1, the kinetic situation for binding of antisense RNA to target RNA appears to reflect RNA-mediated control in vivo (Nordström, K et al. (1988) Gene **72**, 237 - 240). Further structural properties of individual antisense RNAs determine hybridization kinetics in vitro (Persson, C. et al. (1990) EMBO J., **9**, 3767 - 3375) and are linked with biological effects, e.g. plasmid copy numbers (Novick, R.P. (1987) Microbiol. Rev. **51**, 381 - 395; Polsky, B. (1988) Cell, **55**, 929 - 932). In principle these findings are also relevant for other antisense RNA-regulated or -inhibited systems, including eucaryotic ones (Inouye, M. (1988) Gene, 72, 25-34). The biological system in which most of the reported studies with antisense RNA have been conducted include the antisense RNA-mediated inhibition of the human immunodeficiency virus type 1 (HIV-1) replication (Sczakiel, G., Pawlita, M & Kleinheinz, A. (1990) Biochem. Biophys. Res. Comm. 169, 643-651).

We have now investigated kinetic properties in vitro of a HIV-1-directed antisense RNA ($\alpha$Y150) which was shown to inhibit viral replication. For a systematic analysis, individual binding rate constants of successively shortened antisense RNA species derived from $\alpha$Y150 were measured by a new specific experimental method. The results obtained by the newly developed method showed a surprisingly high variation between second order rate constants of subsets of antisense RNA, although chain lengths were similar.

In addition, fast- and slow-hybridizing antisense RNAs were tested for their antiviral activity in a transient HIV-1 replication assay. It was found that the second order rate constants correlated qualitatively with the extent of inhibition of HIV-1 replication.

Therefore, the present invention in general provides a method for identifying fast-hybridizing single-stranded polynucleotides, preferably single-stranded RNA comprising

(a) incubating an unlabelled target polynucleotide with a mixture of labelled polynucleotides containing shortened derivatives of said target polynucleotide;

(b) separating hybridized polynucleotides from non-hybridized polynucleotides after different incubation times of step (a);

(c) analyzing the separated polynucleotides of step (b); and

(d) repeating steps (a) to (c) if necessary.

More particularly the separated polynucleotides of step (b) are preferably analyzed by separating said polynucleotides under denaturing condition and subsequently visualizing the separated polynucleotides.

In a preferred embodiment the target polynucleotide is in excess over said labelled polynucleotides. Particularly the target polynucleotide is incubated with about a 10 to 500 -fold, preferably with about a 50 to 150-fold molar excess of said labelled polynucleotides.

In general, the ratio of length of target polynucleotides to labelled polynucleotides is from about 2:1 to 20:1, preferably from 4:1 to 10:1. A preferred length of the target polynucleotide is about 3000 nucleotides, more preferred 500 - 1000 nucleotides, in particular 200 - 500 nucleotides. A preferred length of the labelled oligonucleotides is about from 15 to 150 nucleotides, preferably from about 50 to 80 nucleotides.

The difference in the particular incubation times is in the range of minutes, preferably between about 1 and 5 minutes. In general, depending on the polynucleotide concentrations and K-values incubation times of up to about 60 minutes can be used.

According to the present application single-stranded polynucleotides are understood as sense polynucleotides, antisense polynucleotides or catalytic antisense polynucleotides. Examples for catalytic antisense polynucleotides are hammerhead ribozymes or hairpin ribozymes. In case of hammerhead ribozymes it is known that hammerhead ribozymes with helix I truncated to about 2 to 5 nucleotides have also cleavage activity and in respect of their strength of inhibition in living cells display no disadvantage by comparison with convential catalytic antisense RNAs or hammerhead ribozymes. This means that RNAs with a 3' antisense arm and a 5' catalytic domain can also be used as inhibitors e.g. against HIV-1. Constructs of this type can therefore also be employed in the in vitro selection method described in the present application, so that the identification of hammerhead ribozymes with improved association rates and inhibitory properties is possible.

Various methods of preparing the starting mixtures of the polynucleotides used in the subsequent selection step are known for the skilled person in the art. One possible method is to synthesize pools of starting polynucleotides in case of DNA by chemical synthesis and in case of RNA by transcription from an appropriate template such as double-stranded DNA or single-stranded DNA/RNA. However, it is pointed out that starting mixtures prepared by other methods known to a skilled person in the art do not limit the selection method as claimed.

The method of analyzing or visualizing the separated polynucleotides depends generally on the label of the target molecule. Any method of labelling of polynucleotides is suitable such as radiolabelling or digoxigenin-labelling which are well known to the skilled person in the art.

Denaturation of polynucleotides is achieved by use of e.g. formamide or urea described e.g. in Rickwood, D. and Hames, B.D. (1985) "Gel electrophoresis of nucleic acids a practical approach" 15-17, JRL Press, Oxford.

In general it is of interest to identify fast-hybridizing nucleic acids because, at least but not limited in the case of long-chain antisense nucleic acids, it is not the binding strength, for example, which is crucial for the biological effect, that is to say the strength of inhibition, but the rate of formation of double strands with the target polynucleotide. Duplex formation in such cases is therefore under kinetic and not thermodynamic control. In this respect the present invention is of particular advantage.

The new method described is applicable for any sequence of interest, for example sequences derived from viruses such as HAV, HBV, HCV, HIV, HSV, HPV, EBV or HCMV or from genes coding for regulatory proteins or enzymes particularly from human or plant genes. Furthermore, the new method is valuable in general for the design of effective high-specific polynucleotides. In therapy of diseases such polynucleotides are useful as inhibitors of e.g. viral replication of gene expression (Uhlmann, E. and Peyman, A. (1990) Chemical Reviews 98, 543). They are also useful in diagnostic applications in detecting specific DNAs or RNAs.

Therefore, another embodiment of the present invention refers to a method of using fast-hybridizing single-stranded polynucleotides identified by the method as described above for the preparation of a diagnostic or therapeutically useful agent. In general, the pharmaceuticals can be prepared as tabletts, dragees, capsules of gelatine, solutions, emulsions or suspensions. The pharmaceuticals can also be produced in the presence of carriers such as lactose, starch or derivatives thereof or stearine. Suitable carries for solutions are water, polyols, saccharose, invert sugar or glucose. The pharmaceuticals can also contain other compounds such as preservatives or stabilizers. A preferred application is the injection of the pharmaceuticals which are preferably formulated in a physiologically acceptable buffer solution such as Hank's solution or Ringer's solution. The preferred dosis is about 0,01 mg/kg to about 50 mg/kg body weight and day.

The application of recombinant genes expressing therapeutically useful single-stranded RNA which has been identified by the claimed method is also part of the invention.

If shorter polynucleotides are going to be used in these process the new method can be applied to identify target regions from which in term shorter oligonucleotides (8-30) can be deduced by calculation of energy values G (Stull et. al. (1992) Nucl. Acids Res. 20, 3501) or by experimental methods.

Another application of high-specific polynucleotides is the use against plant pathogens, in general against virus, bacteria or fungi. In particular, the polynucleotides are useful in inhibiting plant pathogene viruses especially plus and minus strand RNA viruses but also DNA viruses, viroides, virusoides and satellite RNA, for example cucumber mosaic virus, potato virus X and Y, tobacco mosaic virus, water melon mosaic virus, tomato leaf curl virus or tomato spottet wilt virus. In addition, the polynucleotides are useful in applications against physiological factors such as secondary metabolits of plants e.g. coffein or nicotine but also physiological factors in plant compartments of e.g. useful plants such as cereals, maize, soja, rice, field crops, vegetables or fruits.

The invention is now further described in detail in the following figures and examples.

Legends to figures

Figure 1: Plasmid pBS150
Figure 2: Schematic depiction of in vitro selection and identification of fast-hybridizing antisense RNAs.

3

Figure 3: Experimental identification of fast-hybridizing αY150-derived antisense RNAs by electrophoresis with 10 % polyacrylamide gels under denaturing conditions to the scheme shown in Figure 2. Samples were withdrawn from the hybridization reaction mixture at the time points indicated.

Figure 4: Superposition of curves showing the time-dependent decrease of band intensities of distinct educts separated by polyacrylamidegel electrophoresis at time points indicated in Figure 3.

Figure 5: Second order rate constant (k) of the hybridization reaction between αY150-derived antisense RNA species ranging in lengths between 57 and 99 nucleotides (x-axis) and a transcript containing 562 nucleotides of complementary sequence.

Figure 6: Comparison of binding rate constants measured in this work with thermodynamic parameters and the computer-predicted secondary structures.

Figure 7: Comparison of RNAse T1 cleavage reaction.

Figure 8: Comparison of production of HIV-1 after co-transfection with synthesized antisense RNA species and infectious proviral HIV-1 DNA.

EXAMPLES

1. Plasmids for in vitro synthesis of RNAs and target RNA

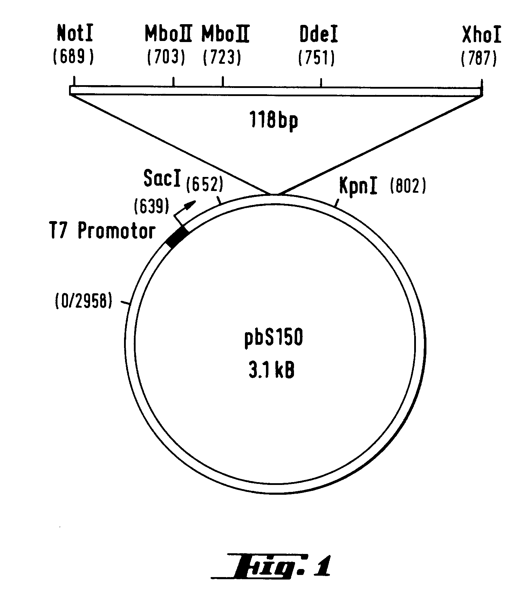

The HIV-1-directed antisense RNAs were transcribed in vitro from plasmid pBS 150 (Fig. 1) which contains two stretches of HIV-1 sequences (17 nts, pos. 5807-5823; 93 nts, pos. 5598-5506; Ratner, L. et al. (1985) Nature **313**, 277-283) in antisense orientiention with respect to a T7 promoter. The pBS150-derived antisense RNA αY150 contains additional polylinker sequences at its 5'-end (32 nts) and at its 3'-end (5 nts). The RNA strand containing complementary sequences to αY150 was transcribed in vitro from plasmid pRC-SR 6 by using T7 polymerase. This sense-transcript contains a 562 nucleotide HIV-1 sequence (clone BH10, pos. 5366-5928; Ratner, L. et al. (1985) excised from plasmid pSR 6 (Rittner, K. & Sczakiel, g. (1991) Nucl. Acids Res., 19, 1421-1426) and polylinker sequences at its 5'-end (74 nts) and at its 3'-end (6 nts).

DNA sequence of the HIV-1 insertion between the restriction enzyme sits NotI and XhoI (Fig. 1)

**NotI**

GCGGCCGCTC ATTGCCACTG TCTTCTTAC TGCCTTGAGG AGGTCTTCGT

CGCTGTCTCC GCTTCTTCCT GCCATAGGAG ATGCCTAAGG CTTTTGTTAT

GAAACAAACT TGGCAATGAC TCGAG

           **XhoI**

RNA sequence of anti sense transcript aY150 (5'→ 3')

GGGCGAAUUG GAGCUCCACC GCGGUGGCGG CCGCUCAUUG CCACUGUCUU

CUUGACUGCC UUGAGGAGGU CUUCGUCGCU GUCUCCGCUU CUUCCUGCCA

UAGGAGAUGC CUAAGGCUUU UGUUAUGAAA CAAACUUGGC

AAUGACUCGA

2. In vitro transcription of RNA

T7 polymerase was used for in vitro transcription of αY150 and SR6 RNAs. Four µg of linearized template DNA (XhoI for αY150 and NotI for SR6) were incubated in a reaction mixture containing 18 Mm $Na_2HPO_4$, 2 Mm $NaH_2PO_4$, 5 Mm NaCl, 20 Mm dithiothreitol, 8 Mm $MgCl_2$, 4 Mm spermidine, and 1 Mm nucleotidetriphosphates in a total volume of 200 µg. Reactions were started by adding 20 U of T7 polymerase and stopped after a 2 hours incubation at 37 °C by adding 300 µl 17 Mm $MgSO_4$ and 20 U DNaseI. After a further incubation for 20 min at 37 °C 750 µl 3 M sodium acetate were added and RNAs were precipitated with ethanol at 0 °C. The pellet was dissolved in 10 Mm Tris/HCL Ph 8.0, 1 Mm EDTA and the RNAs were further purified by gelfiltration (Sephadex[R] G-50, Pharmacia Fine Chemicals, Uppsala, Sweden). The recovery ranged between 40 $\mu$g and 50 $\mu$g RNA. The templates for the smaller $\alpha$Y150-derived antisense RNA species with chain lengths of 60, 67, 76, 82, 88 and 95 nucleotides were generated by PCR according to Schwartz, S. et al. (1990) J. Virol. 64, 2519-2529 with an unique 5'-primer containing the T7 promoter sequence (5'- CCGGATCCAAGCTTTAATACGACTCACTAGGG 3') and 3'-primers (20mers) such that in vitro transcription of RNA terminates at the correct 3'-position. The PCR products were used for in vitro transcription without further subcloning.

3. $^{32}$P-labelling of RNAs

The 5'-ends of in vitro transcribed RNAs (10 ng) were $^{32}$P-labelled by dephosphorylation with calf intestine phosphatase and subsequent rephosphorylation with 5 $\mu$Ci of [$\gamma$-[32P]]-ATP (10.0 mCi/ml, 6000 Ci/mmol) and polynucleotide kinase.

4. Alkaline hydrolysis of $\alpha$Y150-derived antisense RNA

For the production of a random mixture of successively shortened antisense RNAs a modification of an established protocol for alkaline hydrolysis was used (Beijer, B. et al. (1990) Nucl. Acids Res., **18**, 5143-5151). Briefly, 10 ng of 5'-end labelled $\alpha$Y150 RNA dissolved in 20 $\mu$l TE-buffer were added to 500 $\mu$l 500 Mm NaHCO$_3$ and heated to 96 °C for 10 minutes. Hydrolysis was stopped by chilling the reaction mixture and RNAs were desalted by gel filtration (Sephadex[R] G-50, Pharmacia Fine Chemicals) with a buffer containing 10 Mm Tris/Hcl Ph 8.0 and 1 Mm EDTA. In order to make possible native and unique folding of RNA species which does not necessarily happen during the quick chilling on ice water the desalted mixture of RNAs was incubated at 75 °C for 10 minutes and cooled down slowly to 37 °C for renaturation.

5. Assay for selective identification of fast-hybridizing antisense RNAs - analysis of hybridization products

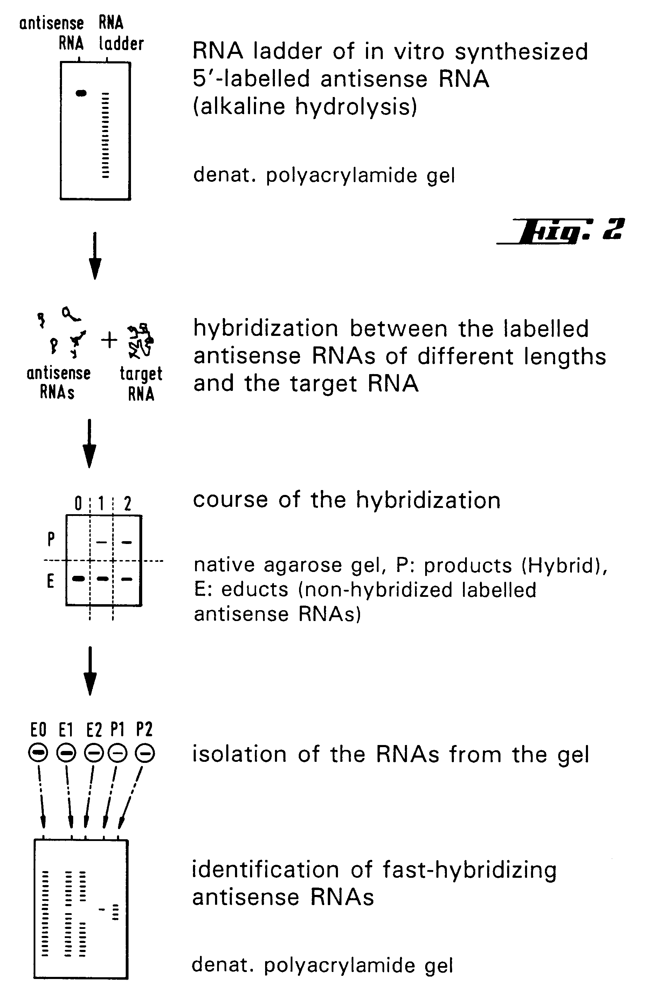

The stepwise experimental procedure is schematically shown in Figure 2. First, one ng of hydrolyzed 5'-labelled $\alpha$Y150 RNA corresponding to 2 x 10$^{-14}$ moles of starting 5'-labelled $\alpha$Y150 RNA (1 x 10$^{-9}$ M) was mixed with 500 ng unlabelled target RNA SR6 (2.2 x 10$^{-12}$ moles, 1.1 x 10$^{-7}$ M) at a final volume of 20 $\mu$l and at 37 °C in a solution containing 100 Mm NaCl, 20 Mm Tris/Hcl Ph 7.4 and 10 Mm MgCl$_2$. After certain time points of incubation, 3 $\mu$l aliquots were withdrawn and transferred into a precooled (0 °C) Eppendorf tube containing 40 $\mu$l stop buffer (20 Mm Tris/Hcl Ph 8.0, 10 Mm EDTA, 0.5 % SDS, 7 M urea, 0.04 % xylenecyanol). Educts and products of the hybridization reaction were separated on 1.2 % agarose gels in 89 Mm Tris-borate buffer Ph 8.3 containing 1 Mm EDTA. Agarose slices containing educts and products respectively were excised and RNAs were eluted by centrifugation of the frozen and thawed (-70 °C/37 °C) gel slizes. RNAs were precipitated with ethanol, redissolved with stop buffer (see above) and analyzed by electrophoresis under denaturing conditions in 10 % polyacrylamide gels containing 7 M urea in 89 Mm Tris-borate Ph 8.3. Gels were fixed in an aqueous solution containing 10 % methanol and 10 % acetic acid, dried and exposed to X-ray film or to image plate analysis for further computer-supported quantitative analysis.

6. Determination of hybridization rates for individual antisense RNA species

For quantitative analysis of band intensities of dried polyacrylamide gels, a self-constructed image plate scanner (Burmester, C. and Schröder, R.R. (1992) Proc. of the European Congress of Electromicrocopy, Eurem 92, Granada, Electronmicrocopy, Vol. 1, 95 - 96,) with a linear characteristic vs $^{32}$P radioactivity was used. Gels were scanned with 37.5 $\mu$m x 37.5 $\mu$m pixelsize. Each lane of the gel was analyzed individually. The data for each band were integrated along a direction perpendicular to the direction of migration. The resulting profiles of intensity against migration distance were displayed using a programme which allowed substraction of background and integration of the individual peaks. Maximal values for each band were taken as a measure for the band intensities. For bands representing certain antisense RNA species at different time points of the hybridization reaction band intensities were plotted against the time axis and a curve for an exponential decay ($I = I_0 \times e^{-k}$) was fitted by non linear regression. The error range for k was derived from the fitting algorithmn as the standard deviation of k.

7. Computer-calculated RNA-structures

The secondary structures of RNAs were calculated with the programme Heidelberg Unix Sequence Analysis Resources (HUSAR) which makes of the secondary structure prediciton algorithmn developed by Zuker and Stiegler (1981) Nucl. Acids Res., 9, 133-148.

8. HIV-1 inhibition studies

For measurements of antisense RNA-mediated inhibition of HIV-1 replication, in vitro synthesized RNAs (120 ng) and infectious proviral HIV-1 DNA (pNL4-3, 40 ng) respectively, were cotransfected by calciumphosphate co-precipitation (Chen. C. & Okayana, H. (1987) Mol. Cell. Biol. 7, 2745-2752) into human SW 480 cells (Leibovitz, A. et al. (1976) Cancer Res. 36, 4562-4569) which were grown semi-confluent in 48 well plates. One day after transfection $1 \times 10^5$ MT-4 cells were added and the final volume was adjusted to 1 ml. Virus replication was measured 4 days after transfection with dilution of cell-free culture supermatants by a commercial HIV-1 antigen ELISA (Organon, Holland) as described in detail elsewhere (Sczakiel, G. (1990) supra). As a control in vitro synthesized CAT-coding RNA was used which had been shown before to have no effect on HIV-1 replication (Rittner, K. & Sczakiel, G. (1991), supra). This assay leads to results similar to those obtained with an earlier described comicroinjection assay except that the error range is significantly smaller for the latter assay.

9. Limited Rnase cleavage of RNA

For Rnase mapping, the 5'ends of RNAs were $^{32}$P-labelled by dephosphorylation with calf intensine phosphatase and subsequent rephosphorylation with [$\gamma$-$^{32}$P]-ATP and polynucleotide kinase. The cleavage reactions were performed in 100 Mm NaCl, 20 Mm Tris/Hcl Ph 7.4, 10 Mm MgCl$_2$ with the following enzyme concentration: Rnase T1, 10 U/ml (Boehringer Mannheim GmbH). The reactions were stopped by adding the sample volume stop buffer (20 Mm Tris/Hcl Ph 8.0, 10 Mm EDTA, 0.5 % SDS, 7 M urea) and chilling on ice. Cleavage products were separated on denaturing polyacrylamide gels (10 %) buffered with 89 Mm Tris-borate Ph 8.3, 2.5 Mm EDTA and 7 M urea.

10. Assay for identifying antisense RNA's with different hybridization rates

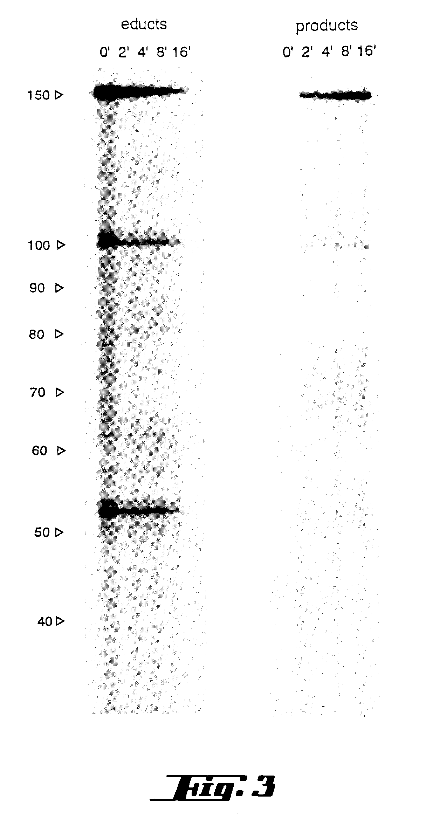

The antisense RNA used in this study ($\alpha$Y150) was directed against coding exons for Tat and Rev in an assay which was used earlier to measure inhibitory effects of in vitro-synthesized HIV-1-directed antisense RNAs (Rittner, K. and Sczakiel, G. (1991) supra. In order to systematically analyze a possible variation of hybridization rates of a set of $\alpha$Y150-derived RNAs which differ in length and, most probably, also in structure we performed the assay which is schematically depicted in Figure 2. A ladder of successively shortened 5'-labelled $\alpha$Y150-derived antisense RNAs was produced by alkaline hydrolysis of 5'-end labelled $\alpha$Y150 RNA (see lane: 0' educts in Figure 3). The resulting mixture of antisense RNAs was incubated with a 110-fold molar excess of complementary unlabelled SR6 RNA in a physiological buffer at 37 °C. This excess of SR6 RNA over $^{32}$P-labelled antisense RNAs was chosen for several reasons: Firstly, it allowed the determination of rate constants according to pseudo first order kinetics. Under those conditions the hybridization rate is not dependent on the concentrations of $^{32}$P-labelled antisense RNA species, i.e. differences in initial concentrations of antisense RNAs do not affect the determination of second order rate constants. Secondly, formation of a defined heteroduplex RNA was favoured versus other possible reactions of labelled antisense RNAs which were observed at equimolar concentrations of a $\alpha$Y150 and SR6 RNA. Lastly, competition among antisense RNAs for complementary RNA strands which could influence the selective determination of k values for individual antisense RNA species was excluded.

The course of the overall hybridization reaction was monitored by separating educts and products by agarosegel electrophoresis. The $^{32}$P-labelled antisense RNAs which were equal to or smaller than 150 nts could be clearly distinguished on agarose gels from product bands, i.e. heteroduplex RNA consisting of one antisense RNA strand and a 562 nts complementary RNA. In order to select and identify individual antisense RNA species contained in early-appearing products of the overall reaction, i.e. fast-hybridizing antisense RNAs, educt bands and product bands were cut out of the agarose gel, RNAs were eluted and analyzed by polyacrylamidegel electrophoresis under denaturing conditions. We did not observe a dependence of elution efficiencies from agarose gels on RNA lengths, however, the elution efficiencies measured for educts, i.e. single-stranded RNAs were significantly greater than those for the double-stranded products. Since the elution efficiency in neither case was dependent on the relative amounts of RNA contained in the eluted bands, the determination of k values was not affected.

## 11. Determination of rate constants for individual antisense RNA species

Presumably, many successive and perhaps easily reversible steps take place between antisense RNA and target RNA before duplex RNAs are formed completely. Since the melting points of the resulting long RNA double strands are high under physiological experimental conditions used in this work, the backward reaction is neglected in the analysis. Therefore, the simplified scheme for the overall hybridization reaction between antisense RNA species ($\alpha RNA_i$) and sense RNA (sRNA) can be described as:

$$\alpha RNA_i + sRNA \rightarrow dsRNA$$

The initial rate of hybridization can be calculated as:

$$v_i = k_{2i}\text{x}[\alpha RNA_i] \times [sRNA] \qquad (eq.1)$$

Since the unlabelled sRNA is in large excess over $\alpha RNA$ and, hence, can be regarded as constant during the reaction, [sRNA] can be included in the rate constant $k_{2i}$:

$$v_i = k_{1i}\text{x}[\alpha RNA_i] \text{ with } k_{1i} = k_{2i}\text{x}[sRNA] \qquad (eq. 2)$$

This is the equation for a reaction of first order for which $k_{1i}$ can be calculated from the time dependence of the binding of $\alpha RNA_i$ to sRNA:

$$[\alpha RNA_i]_t = [\alpha RNA_i]_0 \times e^{-k1it} \qquad (eq. 3)$$

and $k_{1i}$ can be calculated from a computer fit to the data obtained. The value of $k_{2i}$ can be calculated from the relationship: $k_{1i} = [sRNA] \times k_{2i}$

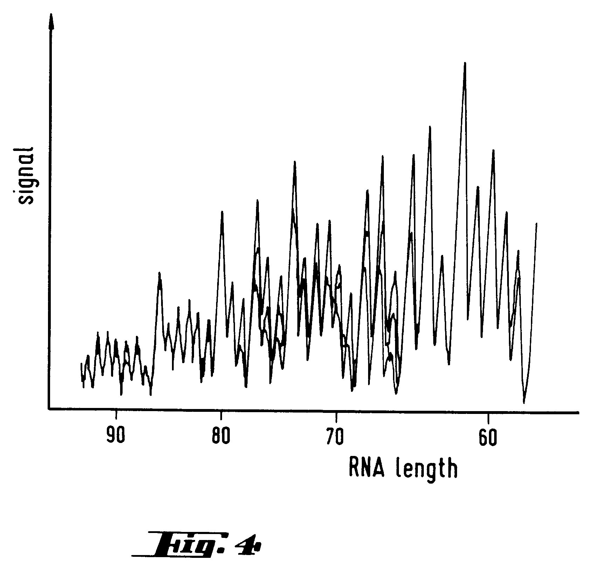

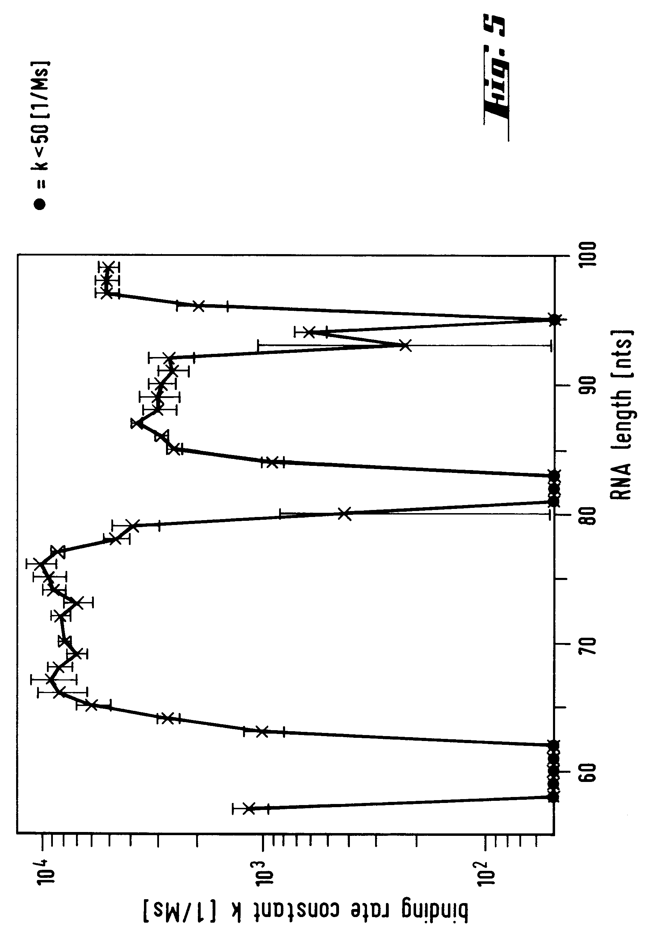

The band pattern of educts and products separated by electrophoresis with 10 % polyacrylamide gels under denaturing conditions clearly demonstrates that disappearing educt bands, i.e. antisense RNA species, correspond to appearing product bands (Figure 3). Further, there are groups of fast-hybridizing antisense RNA species as well as visibly slow-hybridizing species as indicated in Figure 3. For example, fast-hybridizing antisense RNAs range in size between 65 and 78 nts and also occur at 57 nts, 97 nts and 110 nts. Bands containing educts and products of typical hybridization reactions were quantified by using image plate detection and computer-based quantification as described above. For a more detailed analysis we chose the size of 57 to 99 where individual bands could be assigned and differences in hybridization rates were significant. The time-dependent change of educt- and product-signals derived from a representative gel analysis is shown in Figure 4. This analysis shows that educts in certain size ranges (e.g. 66 to 77 nts) hybridize significantly faster ($k = 1 \times 10^4\,M^{-1}s^{-1}$) than those RNA species from other size ranges (e.g. 58-63 or 80-84, Figure 5). For individual antisense RNA species rate constants for the hybridization reaction were calculated by using a fitted curve for the time-dependent decrease of educts (Figure 5). The value for k was calculated for the full length $\alpha Y150$ RNA on the basis of image plate analysis ($k = 1.17 \times 10^4\,M^{-1}s^{-1}$) and compared to values for k determined according to established protocols ($k = 1.30 \times 10^4\,M^{-1}s^{-1}$, Persson, C. et al. (1988) EMBO J. 7, 3279-3288) or to densitometric analysis of band intensities with autoradiographs of polyacrylamidegels ($k = 1.56 \times 10^4\,M^{-1}s^{-1}$). The k value determined by densitometric analysis of autoradiographs with dried polyacrylamidegels is somewhat greater which is due to the overestimation of differences of band intensities by this method. Since, the hybridization rate constant for full length $\alpha Y150$ RNA which had been determined recently to $k = 1.30 \times 10^4 \pm 0.10 \times 10^4\,M^1s^{-1}$ is in agreement with the binding rate constant for $\alpha Y150$ RNA as determined in this work ($k = 1.17 \times 10^4\,[M^{-1}s^{-1}]$), we conclude that k values determined by the assay described here are valid.

## 12. Binding rate constant and RNA structures

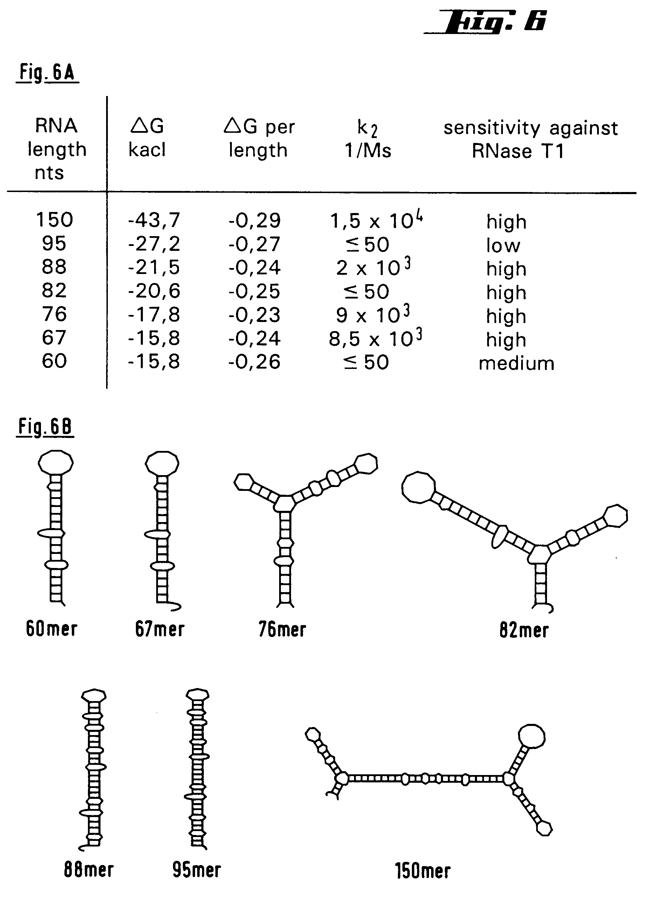

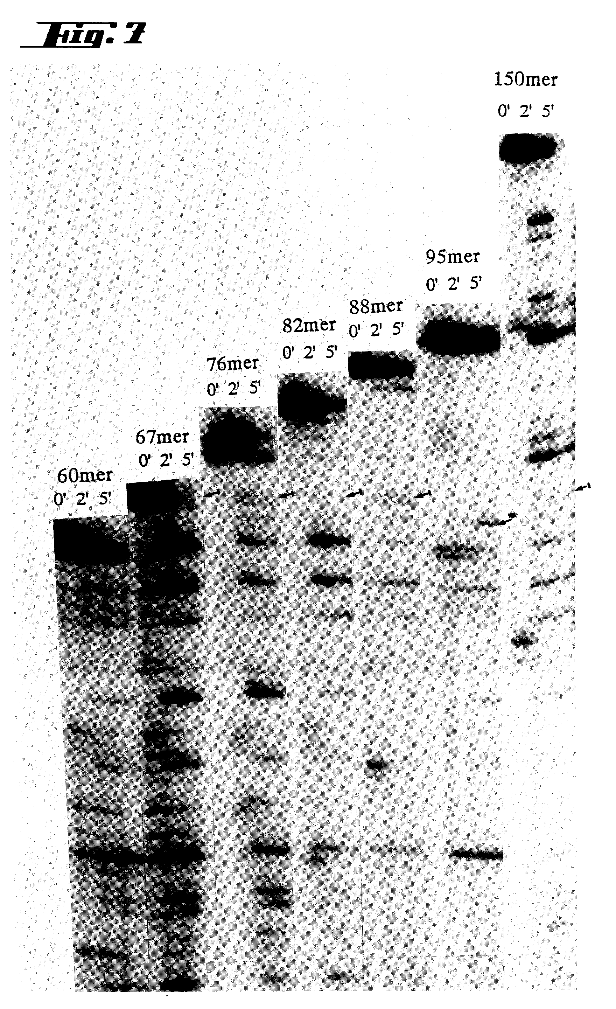

The binding rate constants measured in this work for successively shortened antisense RNAs were compared with the total free energies, the free energy per length, and the computer-predicted secondary structures for each individual molecule (Figure 6). These data do not suggest a correlation between k and any of the structural parameters. The differences between the free energies for the slow-hybridizing antisense RNA species, i.e. -0.29 to -0.25 kcal/nt for the 60mer, 82mer and 95mer and the corresponding parameters for the fast-hydridizing species do not seem to be significant. However, the sensitivity of individual shortened RNAs against Rnase T1 indicates that small k values might correlate with a reduced Rnase sensitivity except for the 82mer which will be discussed later (Figure 6A). Experimental structure analysis of αY150 did not support the predicted secondary structure shown in Figure 6B. In general, mapping of a given RNA molecule by limited cleavage experiments with structure-specific RNases does not enable one to derive an exact model. However, Rnase cleavage patterns may indicate similarities or differences between given RNA molecules. The results of Rnase T1 cleavage reactions with the 60mer, 67mer, 76mer, 82mer, 88mer, 95mer, and αY150 respectively, indicate differences in the 3'portion of the slow-hybridizing 95mer versus the 76mer (fast), 88mer (fast) and αY150 (fast) which also seem to exist versus the fast 67mer (Figure 7). The 60mer, however, lacks this sequence and the 82mer (slow) seems to show the double band labeled in Figure 7, even though significantly weaker than the fasthybridizing species. Thus, the local structures of the sequences in the range of positions 60 to 70 have an effect on the binding rate constants in vitro.

13. Correlation between binding rate constants and the extent of inhibtion in vivo

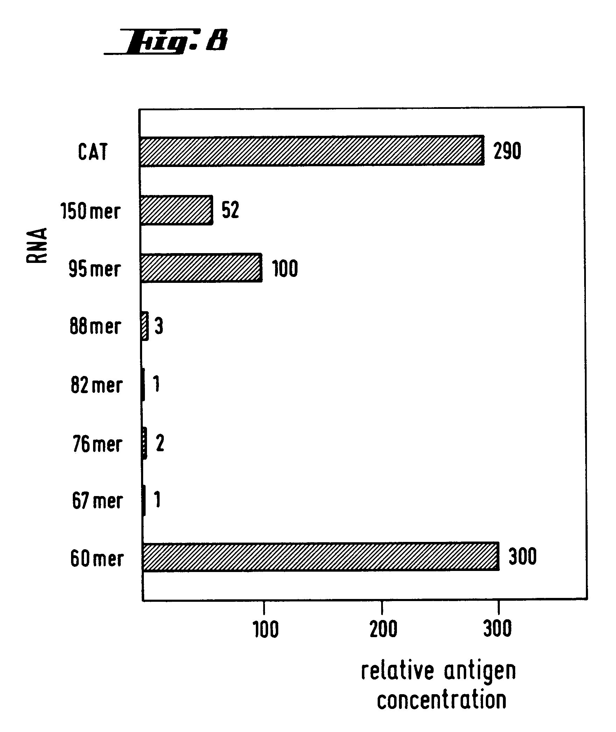

In order to investigate whether the binding rate constants were correlated with the biological effectiveness, we co-transfected in vitro synthesized fast-and slow-hybridizing antisense RNA species with infectious proviral HIV-1 DNA into human cells and measured the resulting production of HIV-1 (Figure 8). Three out of three fast-hybridizing antisene RNAs (67mer, 76mer and 88mer) led to strong inhibition of HIV-1 replication and two out of three slow-hybridizing antisense RNAs (60mer and 95mer) led to a reduct or no significant inhibition respectively. These results indicate that there is a qualitative correlation between the k-values and the inhibitory effectiveness for the αY150-derived antisense RNAs. Possible reasons for the strong inhibition mediated by the "slow" 82mer will be discussed later. It is somewhat surprising that the extent of inhibition in vivo mediated by the full length antisense RNA αY150 is smaller than that of the fast-hybridizing 67mer, 76mer and 88mer respectively.

Conclusion

In the examples an in vitro assay is described in which individual binding rate constants for a series of antisense RNAs to target RNA could be determined in parallel. Measurements with a set of HIV-1-directed antisense RNAs showed that subsets of antisense RNAs with the same 5'-end but successively shortened 3'-ends differ in their hybridization rates with the complementary RNA. A simple length-dependent change of k values was not observed but, groups of fast-hybridizing antisense RNA species with k values in the range of $1 \times 10^4 [M^{-1}s^{-1}]$ (66-77, Figure 5) and $3 \times 10^3 [M^{-1}s^{-1}]$ (85-92, Figure 5) on the one hand and slow-hybridizing ones ($k < 5 \times 10^2 [M^{-1}s^{-1}]$ Figure 5) on the other hand were detected. The hybridization rate for αY150 ($k = 1.3 \times 10^4 \ M^1s^{-1}$) is smaller than one order of magnitude when compared to naturally occuring antisense RNAs with similar lengths (ca $3-10 \times 10^5 \ [M^{-1}s^{-1}]$), summarized in Sczakiel, G., Pawlita, M., Rittner, K. & Homann, M. (1992) Ann. NY Acad. Sci., 660, 268-271). The difference between the binding rate constants determined for fast-hybridizing antisense RNAs in this work and the binding rates for naturally occuring antisense RNAs indicate that further enhancement of the rate of double strand formation between HIV-1 directed antisense RNA and target might be achievable. Thus, kinetic analyses of antisense RNA target interactions is specifically meaningful for further systematic improvements of the antiviral effectiveness of HIV-1 directed antisense and is valuable in general for the design of effective antisense constructs. The transitions from slow-hybridizing to fast-hybridizing antisense RNA species (e.g. pos. 62/65 in Figures 2 and 3) and vice versa (e.g. pos. 78/81 in Figure 3) were found to happen in a small size range. Thus, the question arises whether major structural changes at these chain lengths are linked with the change of k values. For example it is conceivable that the structures of antisense RNAs ranging in length between 66 and 77 share common properties in the sense of similar kinetic characteristics concerning double strand formation, whereas antisense RNAs between 85 and 92 nts in length differ significantly in this respect. For this reason we calculated the k values for all antisense RNAs ranging from 57 to 99 nucleotides on the basis of educt-decrease and compared these with computer-predicted secondary structures calculated according to (Zuker, M. and Stiegler, P. (1981) Nucl. Acids Res. 9, 133 - 148) and corresponding stability parameters for the folded molecules. The results do not indicate a correlation between k values and the structural parameters or corresponding ΔG values respectively. Since computer-aided secondary structure prediction is still not very reliable, we started to compare structures of fast-hybridizing antisense RNAs with slow-hybridizing antisense RNAs experimentally. Limited RNAseT1 cleavage with the antisense RNA species listed in Figure 6A indicated that there was a structural difference in the 3'portion of the slow-hybridizing 95mer versus the other antisense RNAs (Figure 7). For the small and slow 60mer but not the 95mer we cannot exclude that the size of the antisense sequence is too small for efficient inhibition in vivo. One exception seems to be the slow-hybridizing 82mer which led to significant inhibition of HIV-1 replication (Figure 7). In contrast to this, there is only minor experimental evidence for structural differences between the slow 82mer and the fast-hybridizing species which might explain the strong in vivo effects of the 82mer (Figure 7) although the binding rate constant in vitro was small. However, it is known and it can be seen in Figure 7 that in vitro transcription of RNA leads to considerable amounts of shortened and extended transcripts (at least up to three nucleotides). In particular, preparations of the 82mer also contain fast-hybridizing species. For example, the 79mer and 80mer as well als the 84mer and 85mer contained in 82mer preparations might be sufficient to inhibit HIV-1 replication. The experimental conditions for determining HIV-1 replication were chose such that a further comparison of the antiviral effectiveness of the inhibitory antisense RNA species (82mer versus 67mer, 76mer, and 88mer) was not possible.

Therefore, the new method described above is especially very useful in detecting fast-hybridizing RNAs experimentally.

## Claims

1. Method of identifying fast-hybridizing single-stranded polynucleotides comprising
   (a) incubating an unlabelled target polynucleotide with a mixture of labeled polynucleotides containing shortened derivatives of said target polynucleotide;
   (b) separating hybridized polynucleotides from non-hybridized polynucleotides after different incubation times of step (a);
   (c) analyzing the separated polynucleotides of step (b); and
   (d) repeating steps (a) to (c) if necessary.

2. A method according to claim 1 wherein the separated polynucleotides of step (b) are analyzed by:
   (a) separating said polynucleotides under denaturing conditions; and
   (b) visualizing separated polynucleotides of step (a).

3. A method according to claim 1 or 2 wherein said target polynucleotides is in excess over said labelled polynucleotides.

4. A method according to at least one of claims 1 - 3 wherein the ratio of length of target polynucleotides to labelled polynucleotide is from about 2:1 to 20:1.

5. A method according to at least one of claims 1 - 4 wherein the length of said target polynucleotide is about 3000 nucleotides, preferably 500 - 1000 nucleotides, in particular 200 - 500 nucleotides and the length of said labelled polynucleotides is about from 15 to 150 nucleotides, preferably from about 50 to 80 nucleotides.

6. A method according to at least one of claims 1 - 5 wherein the polynucleotide is radiolabelled or digoxigenin-labelled.

7. A method according to at least one of claims 1 - 6 wherein said polynucleotide is a RNA.

8. A method according to claim 7 wherein said RNA is derived from nucleotide sequences of pathogenes, human genes, animal genes or plant genes.

9. A method of using a fast-hybridizing single-stranded polynucleotide identified by a method according to at least one of claims 1 - 8 for inhibiting plant pathogenes or for regulating physiological processes in plants.

10. A method of using fast-hybridizing single-stranded polynucleotides identified by a method according to at least one of claims 1-8 for the preparation of a diagnostic or therapeutically useful agent.

NotI          MboII MboII        DdeI              XhoI
(689)         (703)  (723)       (751)             (787)

118bp

SacI (652)                      KpnI (802)
(639)
T7 Promotor

(0/2958)

pbS150
3.1 kB

Fig. 1

antisense  RNA
RNA  ladder

RNA ladder of in vitro synthesized
5'-labelled antisense RNA
(alkaline hydrolysis)

denat. polyacrylamide gel

*Fig. 2*

antisense     target
RNAs           RNA

hybridization between the labelled
antisense RNAs of different lengths
and the target RNA

0 ┊ 1 ┊ 2

P

E

course of the hybridization

native agarose gel, P: products (Hybrid),
E: educts (non-hybridized labelled
antisense RNAs)

E0  E1  E2  P1  P2

isolation of the RNAs from the gel

identification of fast-hybridizing
antisense RNAs

denat. polyacrylamide gel

*Fig. 3*

signal

RNA length

Fig. 4

● = k<50 [1/Ms]

**Fig. 5**

binding rate constant k [1/Ms]

RNA length [nts]

*Fig. 6*

## Fig. 6A

| RNA length nts | ΔG kacl | ΔG per length | $k_2$ 1/Ms | sensitivity against RNase T1 |
|---|---|---|---|---|
| 150 | -43,7 | -0,29 | $1,5 \times 10^4$ | high |
| 95 | -27,2 | -0,27 | $\leq 50$ | low |
| 88 | -21,5 | -0,24 | $2 \times 10^3$ | high |
| 82 | -20,6 | -0,25 | $\leq 50$ | high |
| 76 | -17,8 | -0,23 | $9 \times 10^3$ | high |
| 67 | -15,8 | -0,24 | $8,5 \times 10^3$ | high |
| 60 | -15,8 | -0,26 | $\leq 50$ | medium |

## Fig. 6B

60mer    67mer    76mer    82mer

88mer    95mer    150mer

## Fig. 7

Fig. 8

## DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,A | NUCLEIC ACIDS RESEARCH vol. 20, no. 13 , 1992 , ARLINGTON, VIRGINIA US pages 3501 - 3508 R.A. STULL ET AL. 'Predicting antisense oligonucleotide inhibitory efficacy' | | C12Q1/68 C12N15/11 C12Q1/70 |
| D,A | GENE. vol. 72 , 1988 , AMSTERDAM NL pages 237 - 240 K. NORDSTRÖM ET AL. 'Translational control by antisense RNA in control of plasmid replication' | | |
| D,A | EMBO JOURNAL. vol. 7, no. 10 , 1988 , EYNSHAM, OXFORD GB pages 3279 - 3288 C. PERSSON ET AL. 'Control of replication of plasmid R1: kinetics of in vitro interaction between the antisense RNA and its target' | | |
| A | AUSUBEL 'Current protocols in molecular biology' , WILEY AND SONS , NEW YORK Part 6, section II * page 6.3.5, column 2, paragraph 3 * * page 6.4.9, column 2, paragraph 3 * | 1 | TECHNICAL FIELDS SEARCHED (Int.Cl.5) C12Q |
| A | EP-A-0 164 876 (ALLIED CORP.) * page 20, paragraph 2 - page 21; example 8 * | 1 | |

The present search report has been drawn up for all claims

| Place of search | Date of completion of the search | Examiner |
|---|---|---|
| THE HAGUE | 8 June 1994 | Molina Galan, E |

EPO FORM 1503 03.82 (P04C01)

SEQUENCE LISTING

(1) GENERAL INFORMATION:

    (i) APPLICANT:
       (A) NAME: Hoechst Aktiengesellschaft
       (B) STREET: -
       (C) CITY: Frankfurt am Main
       (D) STATE: -
       (E) COUNTRY: Federal Republic of Germany
       (F) POSTAL CODE (ZIP): 65926
       (G) TELEPHONE: (069) 305-6031
       (H) TELEFAX: (069) 35-7175
       (I) TELEX: 041234-700 hod

    (ii) TITLE OF INVENTION: Method of identifying fast-hybridizing
       single-stranded polynucleotids

    (iii) NUMBER OF SEQUENCES: 3

    (iv) COMPUTER READABLE FORM:
       (A) MEDIUM TYPE: Floppy disk
       (B) COMPUTER: IBM PC compatible
       (C) OPERATING SYSTEM: PC-DOS/MS-DOS
       (D) SOFTWARE: PatentIn Release #1.0, Version #1.25 (EPO)


(2) INFORMATION FOR SEQ ID NO: 1:

    (i) SEQUENCE CHARACTERISTICS:
       (A) LENGTH: 124 base pairs
       (B) TYPE: nucleic acid
       (C) STRANDEDNESS: single
       (D) TOPOLOGY: linear

    (ii) MOLECULE TYPE: DNA (genomic)

    (iii) HYPOTHETICAL: NO

    (iii) ANTI-SENSE: NO

    (vi) ORIGINAL SOURCE:
       (A) ORGANISM: HIV-1

    (ix) FEATURE:
       (A) NAME/KEY: intron
       (B) LOCATION: 1..124


    (xi) SEQUENCE DESCRIPTION: SEQ ID NO: 1:

GCGGCCGCTC ATTGCCACTG TCTTCTTACT GCCTTGAGGA GGTCTTCGTC GCTGTCTCCG    60

CTTCTTCCTG CCATAGGAGA TGCCTAAGGC TTTTGTTATG AAACAAACTT GGCAATGACT   120

CGAG    124

(2) INFORMATION FOR SEQ ID NO: 2:

    (i) SEQUENCE CHARACTERISTICS:
       (A) LENGTH: 150 base pairs
       (B) TYPE: nucleic acid
       (C) STRANDEDNESS: single
       (D) TOPOLOGY: linear

    (ii) MOLECULE TYPE: mRNA

    (iii) HYPOTHETICAL: NO

```
(iii) ANTI-SENSE: YES

  (vi) ORIGINAL SOURCE:
       (A) ORGANISM: HIV-1

  (ix) FEATURE:
       (A) NAME/KEY: mRNA
       (B) LOCATION: 1..150


  (xi) SEQUENCE DESCRIPTION: SEQ ID NO: 2:

GGGCGAAUUG GAGCUCCACC GCGGUGGCGG CCGCUCAUUG CCACUGUCUU CUUGACUGCC        60

UUGAGGAGGU CUUCGUCGCU GUCUCCGCUU CUUCCUGCCA UAGGAGAUGC CUAAGGCUUU       120

UGUUAUGAAA CAAACUUGGC AAUGACUCGA                                        150

(2) INFORMATION FOR SEQ ID NO: 3:

      (i) SEQUENCE CHARACTERISTICS:
          (A) LENGTH: 32 base pairs
          (B) TYPE: nucleic acid
          (C) STRANDEDNESS: single
          (D) TOPOLOGY: linear

     (ii) MOLECULE TYPE: DNA (genomic)

    (iii) HYPOTHETICAL: NO

    (iii) ANTI-SENSE: NO

     (vi) ORIGINAL SOURCE:
          (A) ORGANISM: Bacteriophage T7

     (ix) FEATURE:
          (A) NAME/KEY: intron
          (B) LOCATION: 1..32


     (xi) SEQUENCE DESCRIPTION: SEQ ID NO: 3:

CCGGATCCAA GCTTTAATAC GACTCACTAG GG                                      32
```